# EUROPEAN PATENT APPLICATION

(11) **EP 4 778 570 A1**
(43) Date of publication of application: **22.07.2026**
(21) Application number: 25152854.3
(22) Date of filing: 20.01.2025
(51) Int. Cl.: A61N 5/10

(54) **RADIATION TREATMENT PLANNING**

(71) Applicant: Siemens Healthineers International AG, 6312 Steinhausen (CH)
(72) Inventor: FALKOVSKIY, Pavel, 00420 Helsinki (FI)
(74) Representative: Mathisen & Macara LLP

(57) **Abstract**

A method used for planning radiation treatment is disclosed. The method comprises, by a processor, accessing information comprising calculated dose and calculated linear energy transfer for a plurality of sub-volumes in a volume in a treatment target, and displaying, on a display device, an image of the treatment target overlayed with a rendering that is based on the calculated doses and the calculated linear energy transfer for the plurality of sub-volumes in the treatment target.

## Description

### TECHNICAL FIELD

The present invention relates to a method used for planning radiation treatment and to a corresponding computer system and non-transitory computer-readable storage medium.

### BACKGROUND

The use of radiation therapy to treat cancer is well known. Typically, radiation therapy involves directing a beam of high energy proton, photon, ion, or electron radiation ("therapeutic radiation") into a target or volume in a treatment target (e.g., a volume that includes a tumor or lesion).

Before a patient is treated with radiation, a treatment plan specific to that patient is developed. The plan defines various aspects of the therapy using simulations and optimizations that may be based on past experiences. In general, the purpose of the treatment plan is to deliver sufficient radiation to the unhealthy tissue while minimizing exposure of surrounding healthy tissue to the radiation.

The planner's goal is to find a solution that is optimal with respect to multiple clinical goals that may be contradictory in the sense that an improvement toward one goal may have a detrimental effect on reaching another goal. For example, a treatment plan that spares the liver from receiving a dose of radiation may result in the stomach receiving too much radiation. These types of tradeoffs lead to an iterative process in which the planner creates different plans to find the one plan that is best suited to achieving the desired outcome.

One challenge is to understand the relationship between radiation dose and linear energy transfer (LET) during radiation therapy planning. LET is a physical quantity that measures the rate at which an ionizing particle transfers energy to material as it passes through it. LET is a function of the particle's charge and energy, as well as the material it passes through. Values of the linear energy transfer can be used to estimate the energy deposited. LET is used to optimize radiation therapies to increase their effectiveness and reduce side effects. Traditionally, radiation dose and LET are visualized separately, which can make it difficult to assess their combined effects on both the target tissue and surrounding healthy tissues.

Up to now, the technical problem of visualizing radiation dose and LET has been addressed through a variety of less integrated methods, such as:
- Sequential Analysis: Clinicians often analyze radiation dose and LET in a sequential manner, looking at separate images or datasets. This process requires mental integration of the two sets of data to understand their combined impact.
- Separate Mapping: Some systems may provide separate color maps or overlays for each parameter. For instance, one image might display the radiation dose in gradients of one color, and another image would show LET in gradients of another color. Clinicians would then need to compare the two maps side by side.

Each of these methods has limitations in terms of ease of use, time efficiency, and the cognitive load on the clinician.

### SUMMARY

In one aspect, the present invention provides a method used for planning radiation treatment, the method comprising, by a processor:
accessing information comprising calculated dose and calculated linear energy transfer for a plurality of sub-volumes in a volume in a treatment target; and
displaying, on a display device an image of the treatment target overlayed with a rendering that is based on the calculated doses and the calculated linear energy transfer for the plurality of sub-volumes in the treatment target.

Embodiments of the invention seek to improve upon known methods of visualizing radiation dose and linear energy transfer by providing a more intuitive and immediate way to visualize and interpret the critical parameters of radiation therapy.

The method may further comprise associating attribute values to elements of the rendering corresponding to the calculated doses and the calculated linear energy transfers; and displaying the elements according to the attribute values. The attribute values may be values of color and intensity.

At least a portion of the calculated dose may be displayed in a first color (e.g. red) and at least a portion of the calculated linear energy transfer may be displayed in a second color (e.g. green), different to the first color. This provides an easily recognisable way of showing both dose and LET in a single image.

The portion of the calculated dose displayed in the first color may be displayed with an intensity that is dependent on the value of the dose and the portion of the calculated linear energy transfer displayed in the second color may be displayed with an intensity that is dependent on the value of the linear energy transfer. This provides an easily recognisable way of showing the value of the dose and LET in a single image.

Sub-volumes of the treatment target that are calculated to receive dose and linear energy transfer may be displayed in a third color (e.g. yellow), different to the first color and the second color. The portion of the calculated dose and linear energy transfer that is displayed in the third color is displayed with an intensity that is dependent on the value of the dose and the linear energy transfer. This may allow intuitive identification of areas receiving intense treatment.

Alternatively, at least a portion of the calculated dose is displayed in a color selected in dependence of the dose value, and at least a portion of the calculated linear energy transfer is displayed in an intensity selected in dependence of the LET value. This provides an easily recognisable way of showing both dose and LET in a single image.

The image of the treatment target may be a CT scan, although other image and scan types may also be used with the invention.

The method may include displaying, on the display device, a graphical user interface that allows a user to adjust the dose and/or the linear energy transfer, recalculating the dose and/or the linear energy transfer values, and displaying, on the display device, the image of the treatment target overlayed with a rendering that is based on the recalculated dose and/or the recalculated linear energy transfer. An iterative process of varying dose and/or the linear energy transfer may therefore be preformed that allows a user to visualize the effects of varying dose and/or the linear energy transfer.

In another aspect, the present invention provides a computer system as defined in the claims.

In a further aspect, the present invention provides a non-transitory computer-readable storage medium having computer-executable instructions for causing a computer system to perform the method, as defined in the claims.

Embodiments of the invention may integrate with existing medical imaging platforms and overlay the radiation dose and LET rate information onto patient scans. The radiation dose may be represented by varying intensities of one color channel (e.g. the red color channel), while the LET is represented by a different color channel (e.g. the green color channel). When regions have both high dose and high LET, the colors combine to form another color (e.g. yellow), allowing for intuitive identification of areas receiving intense treatment. This method may enhance the clinician's ability to evaluate and adjust treatment plans.

Known solutions typically require separate analysis or visual representation for radiation dose and LET. Embodiments integrate both parameters into one image. Advantageously, this integrated approach simplifies the interpretation process, reduces cognitive workload, and may decrease the likelihood of errors that could arise from trying to mentally merge two separate datasets. Embodiments allow a user to evaluate more efficiently quality of two different but related volumetric distributions: dose distribution and LET distribution. Those two volumetric distributions are combined into one distribution.

In one embodiment, the use of universally understood color blending (e.g. red, green, yellow) simplifies the learning curve compared to interpreting separate color maps or numerical data.

The system's intuitive design can shorten training time for medical staff and enhance the understanding of complex dosimetric information.

### BRIEF DESCRIPTION OF DRAWINGS

The accompanying drawings, which are incorporated in and form a part of this specification and in which like numerals depict like elements, illustrate embodiments of the present disclosure and, together with the detailed description, serve to explain the principles of the disclosure.
Figure 1 is a block diagram of an example of a computer system upon which the embodiments described herein may be implemented.
Figure 2 is a block diagram illustrating an example of an automated radiation therapy treatment planning system in embodiments according to the present invention.
Figure 3 illustrates a knowledge-based planning system in embodiments according to the present invention.
Figure 4 is a block diagram showing selected components of a radiation therapy system upon which embodiments according to the present invention can be implemented.
Figures 5 is a flowchart of an example of computer-implemented operations for planning radiation treatment in embodiments according to the present invention.
Figure 6 is an example of a display on a display device and used for planning radiation treatment in embodiments according to the present invention.
Figure 7 is another example of a display on a display device and used for planning radiation treatment in embodiments according to the present invention.

### DETAILED DESCRIPTION

Reference will now be made in detail to the various embodiments of the present disclosure, examples of which are illustrated in the accompanying drawings. While described in conjunction with these embodiments, it will be understood that they are not intended to limit the disclosure to these embodiments. On the contrary, the disclosure is intended to cover alternatives, modifications and equivalents, which may be included within the scope of the disclosure as defined by the appended claims. Furthermore, in the following detailed description of the present disclosure, numerous specific details are set forth in order to provide a thorough understanding of the present disclosure. However, it will be understood that the present disclosure may be practiced without these specific details. In other instances, well-known methods, procedures, components, and circuits have not been described in detail so as not to unnecessarily obscure aspects of the present disclosure.

Some portions of the detailed descriptions that follow are presented in terms of procedures, logic blocks, processing, and other symbolic representations of operations on data bits within a computer memory. These descriptions and representations are the means used by those skilled in the data processing arts to most effectively convey the substance of their work to others skilled in the art. In the present application, a procedure, logic block, process, or the like, is conceived to be a self-consistent sequence of steps or instructions leading to a desired result. The steps are those utilizing physical manipulations of physical quantities. Usually, although not necessarily, these quantities take the form of electrical or magnetic signals capable of being stored, transferred, combined, compared, and otherwise manipulated in a computer system. It has proven convenient at times, principally for reasons of common usage, to refer to these signals as transactions, bits, values, elements, symbols, characters, samples, pixels, or the like.

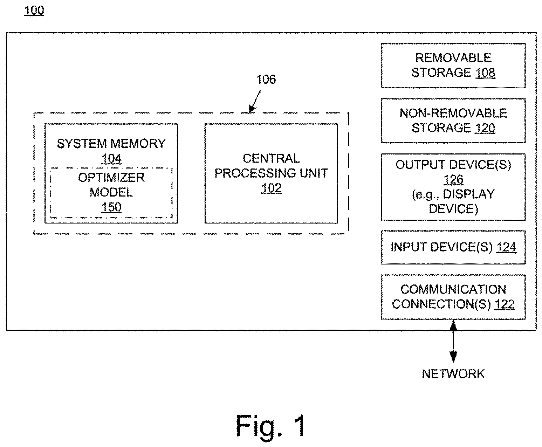

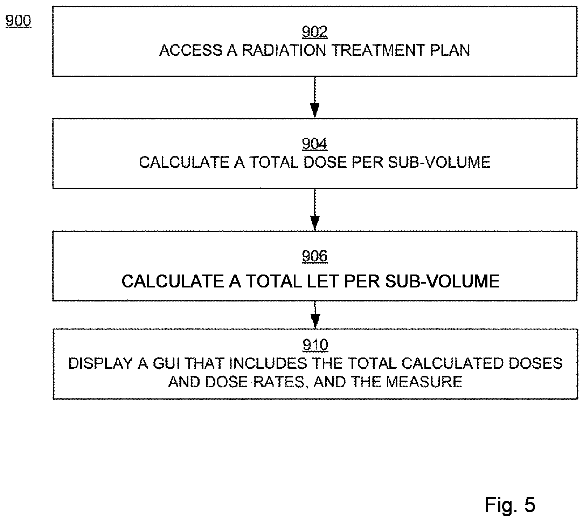

It should be borne in mind, however, that all of these and similar terms are to be associated with the appropriate physical quantities and are merely convenient labels applied to these quantities. Unless specifically stated otherwise as apparent from the following discussions, it is appreciated that throughout the present disclosure, discussions utilizing terms such as "accessing," "generating," "representing," "applying," "indicating," "storing," "using," "adjusting," "including," "computing," "calculating," "determining," "visualizing," "displaying," "rendering," "associating," "binning," or "rounding," or the like, refer to actions and processes (e.g., the flowchart of Figure 5) of a computer system or similar electronic computing device or processor (e.g., the computer system 100 of Figure 1). The computer system or similar electronic computing device manipulates and transforms data represented as physical (electronic) quantities within the computer system memories, registers or other such information storage, transmission or display devices.

The discussion to follow includes terms such as "dose," "energy," etc. Unless otherwise noted, a value is associated with each such term. For example, a dose has a value and can have different values. For simplicity, the term "dose" may refer to a value of a dose, for example, unless otherwise noted or apparent from the discussion.

Portions of the detailed description that follows are presented and discussed in terms of methods. Although steps and sequencing thereof are disclosed in figures herein (e.g., Figure 5) describing the operations of those methods, such steps and sequencing are examples only. Embodiments are well suited to performing various other steps or variations of the steps recited in the flowcharts of the figures herein, and in a sequence other than that depicted and described herein.

Embodiments described herein may be discussed in the general context of computer-executable instructions residing on some form of computer-readable storage medium, such as program modules, executed by one or more computers or other devices. By way of example, and not limitation, computer-readable storage media may comprise non-transitory computer storage media and communication media. Generally, program modules include routines, programs, objects, components, data structures, etc., that perform particular tasks or implement particular abstract data types. The functionality of the program modules may be combined or distributed as desired in various embodiments.

Computer storage media includes volatile and nonvolatile, removable and non-removable media implemented in any method or technology for storage of information such as computer-readable instructions, data structures, program modules or other data. Computer storage media includes, but is not limited to, random access memory (RAM), read only memory (ROM), electrically erasable programmable ROM (EEPROM), flash memory or other memory technology, compact disk ROM (CD-ROM), digital versatile disks (DVDs) or other optical storage, magnetic cassettes, magnetic tape, magnetic disk storage or other magnetic storage devices, or any other medium that can be used to store the desired information and that can accessed to retrieve that information.

Communication media can embody computer-executable instructions, data structures, and program modules, and includes any information delivery media. By way of example, and not limitation, communication media includes wired media such as a wired network or direct-wired connection, and wireless media such as acoustic, radio frequency (RF), infrared and other wireless media. Combinations of any of the above can also be included within the scope of computer-readable media.

Figure 1 shows a block diagram of an example of a computer system 100 upon which the embodiments described herein may be implemented. In its most basic configuration, the system 100 includes at least one processing unit 102 and memory 104. This most basic configuration is illustrated in Figure 1 by dashed line 106. The system 100 may also have additional features and/or functionality. For example, the system 100 may also include additional storage (removable and/or non-removable) including, but not limited to, magnetic or optical disks or tape. Such additional storage is illustrated in Figure 1 by removable storage 108 and non-removable storage 120. The system 100 may also contain communications connection(s) 122 that allow the device to communicate with other devices, e.g., in a networked environment using logical connections to one or more remote computers.

The system 100 also includes input device(s) 124 such as keyboard, mouse, pen, voice input device, touch input device, etc. Output device(s) 126 such as a display device, speakers, printer, etc., are also included. A display device may be, for example, a cathode ray tube display, a light-emitting diode display, or a liquid crystal display.

In the example of Figure 1, the memory 104 includes computer-readable instructions, data structures, program modules, and the like associated with an "optimizer" model 150. However, the optimizer model 150 may instead reside in any one of the computer storage media used by the system 100, or may be distributed over some combination of the computer storage media, or may be distributed over some combination of networked computers. The functionality of the optimizer model 150 is described below.

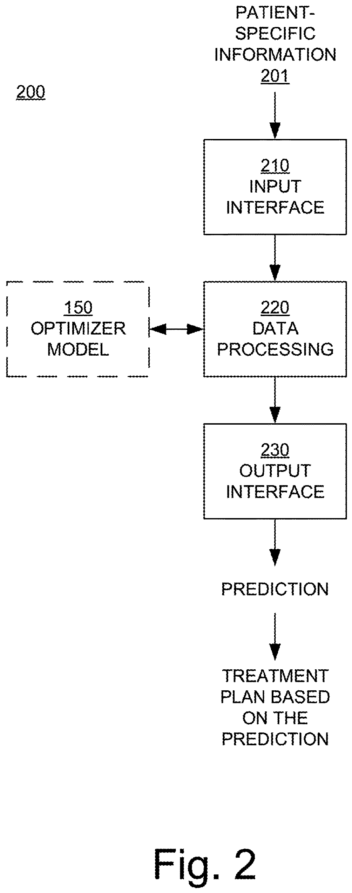

Figure 2 is a block diagram illustrating an example of an automated radiation therapy treatment planning system 200 in embodiments according to the present invention. The system 200 includes an input interface 210 to receive patient-specific information (data) 201, a data processing component 220 that implements the optimizer model 150, and an output interface 230. The system 200 in whole or in part may be implemented as a software program, hardware logic, or a combination thereof on/using the computer system 100 (Figure 1).

In the example of Figure 2, the patient-specific information is provided to and processed by the optimizer model 150. In embodiments, the optimizer model 150 yields a prediction result, and a treatment plan based on the prediction result can then be generated.

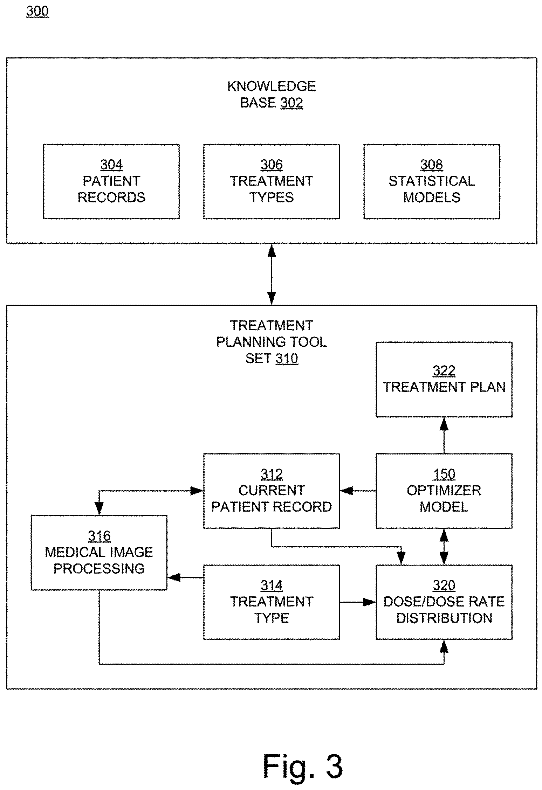

Figure 3 illustrates a knowledge-based planning system 300 in embodiments according to the present invention. In the example of Figure 3, the system 300 includes a knowledge base 302 and a treatment planning tool set 310. The knowledge base 302 includes patient records 304 (e.g., radiation treatment plans), treatment types 306, and statistical models 308. The treatment planning tool set 310 in the example of Figure 3 includes a current patient record 312, a treatment type 314, a medical image processing module 316, the optimizer model (module) 150, a dose distribution module 320, and a final radiation treatment plan 322.

The treatment planning tool set 310 searches through the knowledge base 302 (through the patient records 304) for prior patient records that are similar to the current patient record 312. The statistical models 308 can be used to compare the predicted results for the current patient record 312 to a statistical patient. Using the current patient record 312, a selected treatment type 306, and selected statistical models 308, the tool set 310 generates a radiation treatment plan 322.

More specifically, based on past clinical experience, when a patient presents with a particular diagnosis, stage, age, weight, sex, co-morbidities, etc., there can be a treatment type that is used most often. By selecting the treatment type that the planner has used in the past for similar patients, a first-step treatment type 314 can be chosen. Patient outcomes, which can include normal tissue complication probability as a function of dose rate and patient-specific treatment-type outcomes (e.g., local recurrent failure, and overall survival as a function of a dose and/or dose rate) can be included in the treatment planning process. The medical image processing module 316 provides automatic contouring and automatic segmentation of two-dimensional cross-sectional slides (e.g., from any imaging modality such as, but not limited to, computed tomography (CT), positron emission tomography-CT, magnetic resonance imaging, and ultrasound) to form a three-dimensional (3D) image using the medical images in the current patient record 312. Dose distribution maps, LET distribution maps, and dose rate distribution maps are calculated by the dose, LET and dose rate distribution module 320, which may utilize the optimizer model 150.

The discussion to follow refers to beams, volumes, doses, dose rates, LET and other elements or values. The discussion below is in the context of modeled elements and calculated values in the treatment planning tool set 310 and the optimizer model 150 (Figure 3), unless otherwise noted or made clear in the discussion.

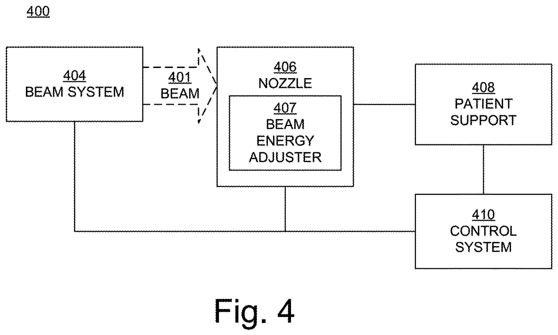

Figure 4 is a block diagram showing selected components of a radiation therapy system 400 upon which embodiments according to the present invention can be implemented. In the example of Figure 4, the system 400 includes a beam system 404 and a nozzle 406.

The beam system 404 generates and transports a beam 401. The beam 401 can be a proton beam, electron beam, photon beam, ion beam, or atom nuclei beam (e.g., carbon, helium, and lithium). In embodiments, depending on the type of beam, the beam system 404 includes components that direct (e.g., bend, steer, or guide) the beam system in a direction toward and into a nozzle 406. In embodiments, the radiation therapy system may include one or more multileaf collimators (MLCs); each MLC leaf can be independently moved back-and-forth by the control system 410 to dynamically shape an aperture through which the beam can pass, to block or not block portions of the beam and thereby control beam shape and exposure time. The beam system 404 may also include components that are used to adjust (e.g., reduce) the beam energy entering the nozzle 406.

The nozzle 406 is used to aim the beam toward various locations (a volume in a treatment target) (e.g., a volume in a patient) supported on the patient support device 408 (e.g., a chair or table) in a treatment room. A volume in a treatment target may be an organ, a portion of an organ (e.g., a volume or region within the organ), a tumor, diseased tissue, or a patient outline. A volume in a treatment target may include both unhealthy tissue (e.g., a tumor) and healthy tissue. A volume in a treatment target may be divided (virtually) into a number of voxels. A sub-volume can include a single voxel or multiple voxels.

The nozzle 406 may be mounted on or a part of a gantry that can be moved relative to the patient support device 408, which may also be moveable. In embodiments, the beam system 404 is also mounted on or is a part of the gantry. In another embodiment, the beam system is separate from (but in communication with) the gantry.

The control system 410 of Figure 4 receives and implements a prescribed radiation treatment plan. In embodiments, the control system 410 includes a computer system having a processor, memory, an input device (e.g., a keyboard), and perhaps a display in well-known fashion. The control system 410 can receive data regarding operation of the system 400. The control system 410 can control parameters of the beam system 404, nozzle 406, and patient support device 408, including parameters such as the energy, intensity, direction, size, and/or shape of the beam, according to data it receives and according to the prescribed radiation treatment plan.

As noted above, the beam 401 entering the nozzle 406 has a specified energy. Thus, in embodiments according to the present disclosure, the nozzle 406 includes one or more components that affect (e.g., decrease, modulate) the energy of the beam. The term "beam energy adjuster" is used herein as a general term for a component or components that affect the energy of the beam, in order to control the range of the beam (e.g., the extent that the beam penetrates into a target), to control the dose delivered by the beam, and/or to control the depth-dose curve of the beam, depending on the type of beam. For example, for a proton beam or an ion beam that has a Bragg peak, the beam energy adjuster can control the location of the Bragg peak in the volume in a treatment target. In various embodiments, the beam energy adjuster 407 includes a range modulator, a range shifter, or both a range modulator and a range shifter.

In radiation therapy techniques in which the intensity of the particle beam is either constant or modulated across the field of delivery, such as in intensity modulated radiation therapy (IMRT) and intensity modulated particle therapy (IMPT), beam intensity is varied across each treatment region (volume in a treatment target) in a patient. Depending on the treatment modality, the degrees of freedom available for intensity modulation include beam shaping (collimation), beam weighting (spot scanning), and angle of incidence (which may be referred to as beam geometry). These degrees of freedom lead to an effectively infinite number of potential treatment plans, and therefore consistently and efficiently generating and evaluating high-quality treatment plans is beyond the capability of a human and relies on the use of a computer system, particularly considering the time constraints associated with the use of radiation therapy to treat ailments like cancer, as well as the large number of patients that are undergoing or need to undergo radiation therapy during any given time period.

The beam 401 can have virtually any regular or irregular cross-sectional (e.g., beam's eye view) shape. For example, the shape of the beam 401 can be defined using an MLC that blocks a portion or portions of the beam. Different beams can have different shapes.

In operation, in embodiments, the beam segments are delivered sequentially. For example, a first beam segment is delivered to the volume in a treatment target (turned on) and then turned off, then a second beam segment is turned on then off, and so on. Each beam segment may be turned on for only a fraction of a second (e.g., on the order of milliseconds).

A single beam may be used and applied from different directions and in the same plane or in different planes. Alternatively, multiple beams may be used, in the same plane or in different planes. The directions and/or numbers of beam can be varied over a number of treatment sessions (that is, fractionated in time) so that a uniform dose is delivered across the volume in the treatment target. The number of beams delivered at any one time depends on the number of gantries or nozzles in the radiation treatment system (e.g., the radiation treatment system 400 of Figure 4) and on the treatment plan.

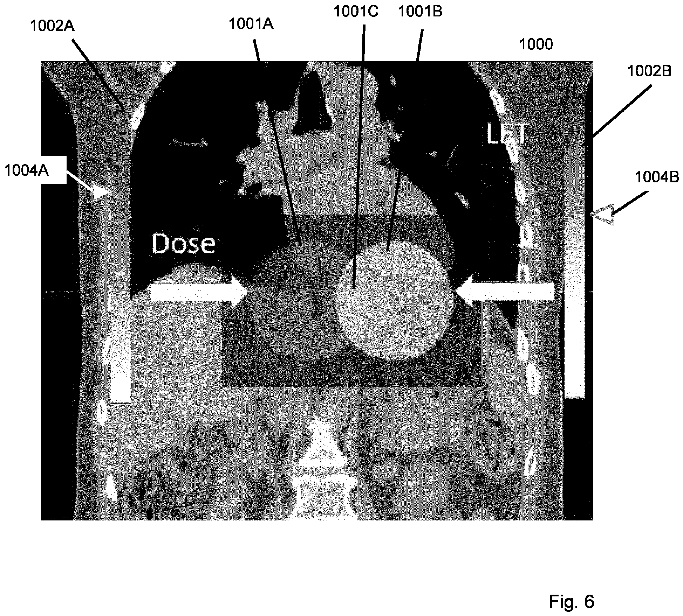

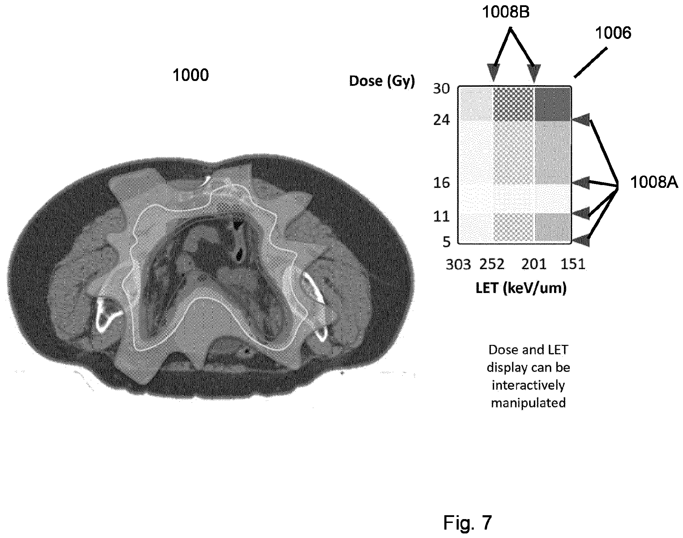

Figure 5 is a flowchart 900 that is an example of a computer-implemented method for planning radiation treatment in embodiments according to the present invention. The flowchart 900 can be implemented as computer-executable instructions (e.g., the optimizer model 150 of Figure 1) residing on some form of computer-readable storage medium (e.g., in memory of the computer system 100 of Figure 1). In this embodiment, as a result of the disclosed method, a GUI is generated and displayed. The GUI visualizes, in a single rendering, calculated doses (e.g., total calculated doses) and calculated LETs in a treatment target. Examples of a GUI in accordance with the present invention are provided in Figure 6 and 7.

With reference now to Figure 5, in block 902, a radiation treatment plan is accessed from computer system memory. The radiation treatment plan includes, for example, a number of beams to be directed at and into a volume in a treatment target, directions of the beams, and a range of LETs for each of the beams.

In block 904, a dose (e.g., total dose) per sub-volume is calculated using the number and directions of the beams and the range of LETs.

In block 906, a LET per sub-volume is calculated using the number and directions of the beams and the range of LETs.

In block 910, a GUI that includes a rendering (e.g., a visual display) that is based on the calculated doses and the calculated LETs is displayed on the display device 126 (Figure 1).

In the example of Figure 6, the GUI 1000 includes a rendering showing a patient image (e.g. a CT scan of a region of the patient). The rendering includes an overlay of the radiation dose (1001A) and LET (1001B) information onto the patient scan. The radiation dose may be represented by varying intensities of one color channel (e.g. the red color channel), while the LET is represented by a different color channel (e.g. the green color channel). When regions have both high dose and high LET, the colors combine (1001C) to form another color (e.g. yellow), allowing for intuitive identification of areas receiving intense treatment.

A color key 1002A, 1002B may be included in the GUI 1000 to associate the colors in the rendering with dose (key part 1002A) and LET (key part 1002B). In this example one color such as red is used for dose and a different color such as green is used for LET. The key 1002A, 1002B may also associate the intensity (or lightness or saturation) of the color such as red with a particular dose amount and associate the intensity (or lightness or saturation) of the color such as green with a particular LET amount.

The rendering can be manipulated using the key 1002A, 1002B, by using pointers 1004A, 1004B to select different levels of dose and different LET amounts to be rendered in the GUI 1000. A user can interactively change the positions of the pointers 1004A, 1004B on either or both of the dose (pointer 1004A) and LET (pointer 1004B). The adjusted value of dose and/or LET is then applied to the treatment plan, which is revised using the adjusted value of dose and/or LET. The GUI is displayed that includes a rendering (e.g., a visual display) that is based on newly calculated doses and/or LETs. The rendering shows the dose and LET overlaid on the patient image, as in Fig. 6, (but with updated values).

In the example of Figure 6, the GUI 1000 rendering includes the overlay of the radiation dose (1001A) which has a single intensity and LET (1001B) which has a single intensity. The combined colors (1001C) also have a single intensity. In practice, the radiation dose (1001A), LET (1001B) and the combined colors (1001C) will have varying intensities in different regions of the patient image. That is, the intensity of the green, red and yellow colors in the example will vary for different regions of the patient image in accordance with the dose, LET and combined dose/LET.

In the example of Figure 7, the GUI 1000 includes a rendering showing a patient image (e.g. a CT scan of a region of the patient). The rendering includes an overlay of the radiation dose and LET information onto the patient scan. The radiation dose is represented by varying color. The one color may represent a low dose range and another color may represent a high dose range. Intermediate dose ranges may be represented by other colors. In the example shown, a low dose range (e.g. 5-11Gy) may be indicated by green, with yellow, orange and red colors indicating progressively higher doses (e.g. 11-16Gy, 16-24Gy and 24-30Gy, respectively). The LET is represented by varying intensity. A low intensity may represent a high LET range (e.g. 303-252 keV/µm and a higher intensity may represent a low LET range (e.g. 201-151 keV/µm). An intermediate dose range may be represented by other intensities (e.g. 252-201 keV/µm). In the example shown, progressively higher intensities indicate progressively lower LET values. The rendering conveys the dose and LET at any given position by the color at the position (for dose) and by the intensity of the color at the position (for LET).

A color key 1006 may be included in the GUI 1000 of Fig. 7 to associate the colors in the rendering with dose and to associate the intensities in the rendering with LET. The rendering can be manipulated using the key 1006, by using pointers 1008A, 1008B to select different levels of dose and different LET amounts to be rendered in the GUI 1000. A user can interactively change the positions of the pointers 1008A, 1008B on either or both of the dose (pointer 1008A) and LET (pointer 1008B). The adjusted value of dose and/or LET is then applied to the treatment plan, which is revised using the adjusted value of dose and/or LET. The GUI is displayed that includes a rendering (e.g., a visual display) that is based on newly calculated doses and/or LETs. The rendering shows the dose and LET overlaid on the patient image, as in Fig. 7, (but with updated values).

The GUI 1000 shows a CT scan with the described color channel overlays. The regions of interest, such as a tumor receiving radiation therapy, can be easily discerned by clinicians, optimizing treatment efficacy and safety. This visualization allows for an immediate and clear understanding of the treatment area, enabling clinicians to make informed decisions quickly.

In addition to those benefits, a GUI facilitates treatment planning by allowing a planner to readily visualize key elements of a proposed treatment plan (e.g., the LET per sub-volume), to readily visualize the effects on those elements of changes to the proposed plan, and to readily visualize a comparison between different plans.

Although the subject matter has been described in language specific to structural features and/or methodological acts, it is to be understood that the subject matter defined in the appended claims is not necessarily limited to the specific features or acts described above. Rather, the specific features and acts described above are disclosed as example forms of implementing the claims.

## Claims

1. A method used for planning radiation treatment, the method comprising, by a processor:
accessing information comprising calculated dose and calculated linear energy transfer for a plurality of sub-volumes in a volume in a treatment target; and
displaying, on a display device, an image of the treatment target overlayed with a rendering that is based on the calculated doses and the calculated linear energy transfer for the plurality of sub-volumes in the treatment target.

2. The method of claim 1, wherein the method further comprises:
associating attribute values to elements of the rendering corresponding to the calculated doses and the calculated linear energy transfers; and
displaying the elements according to the attribute values.

3. The method of Claim 2, wherein the attribute values are values of color and intensity.

4. The method of claim 1, 2 or 3, wherein at least a portion of the calculated dose is displayed in a first color and at least a portion of the calculated linear energy transfer is displayed in a second color, different to the first color.

5. The method of claim 4, wherein the portion of the calculated dose displayed in the first color is displayed with an intensity that is dependent on the value of the dose and the portion of the calculated linear energy transfer displayed in the second color is displayed with an intensity that is dependent on the value of the linear energy transfer.

6. The method of claim 4 or 5, wherein sub-volumes of the treatment target that are calculated to receive dose and linear energy transfer are displayed in a third color, different to the first color and the second color.

7. The method of claim 6, wherein the portion of the calculated dose and linear energy transfer displayed in the third color is displayed with an intensity that is dependent on the value of the dose and the linear energy transfer.

8. The method of claim 1, 2 or 3, wherein at least a portion of the calculated dose is displayed in a color selected in dependence of the dose value, and at least a portion of the calculated linear energy transfer is displayed in an intensity selected in dependence of the LET value.

9. The method of any one of claims 1 to 8, wherein the image of the treatment target is a CT scan.

10. The method of any one of claims 1 to 9, wherein the method includes displaying, on the display device, a graphical user interface that allows a user to adjust the dose and/or the linear energy transfer, recalculating the dose and/or the linear energy transfer values, and displaying, on the display device, the image of the treatment target overlayed with a rendering that is based on the recalculated dose and/or the recalculated linear energy transfer.

11. A computer system, comprising:
a processor;
a display device coupled to the processor; and
memory coupled to the processor and comprising instructions that, when executed, cause the processor to perform the method of any one of claims 1 to 10.

12. A non-transitory computer-readable storage medium having computer-executable instructions for causing a computer system to perform the method of any one of claims 1 to 10.
